# EUROPEAN PATENT APPLICATION

(11) **EP 3 808 834 A1**
(43) Date of publication of application: **21.04.2021**
(21) Application number: 19818797.3
(22) Date of filing: 06.06.2019
(51) Int. Cl.: C12M 1/34

(54) **INFORMATION PROCESSING DEVICE, DERIVATION METHOD, AND DERIVATION PROGRAM**

(30) Priority: 13.06.2018 JP 2018112878
(71) Applicant: FUJIFILM Corporation, Tokyo 106-8620 (JP)
(72) Inventor: MURAKAMI, Yuta, Ashigarakami-gun, Kanagawa 258-8577 (JP); FUJINAMI, Tatsuya, Ashigarakami-gun, Kanagawa 258-8577 (JP); MATSUBARA, Kenta, Ashigarakami-gun, Kanagawa 258-8577 (JP); SATO, Katsuko, Ashigarakami-gun, Kanagawa 258-8577 (JP); YAMAMOTO, Ayako, Ashigarakami-gun, Kanagawa 258-8577 (JP); TAKAYAMA, Hidetoshi, Ashigarakami-gun, Kanagawa 258-8577 (JP); WAKUI, Takashi, Ashigarakami-gun, Kanagawa 258-8577 (JP); AIBA, Satoshi, Ashigarakami-gun, Kanagawa 258-8577 (JP); KIKUCHI, Shinichi, Ashigarakami-gun, Kanagawa 258-8577 (JP); NAGASE, Masaya, Ashigarakami-gun, Kanagawa 258-8577 (JP); LING, Xiao, Ashigarakami-gun, Kanagawa 258-8577 (JP)
(74) Representative: Klunker IP Patentanwälte PartG mbB
(86) International application number: PCT/JP2019/022561
(87) International publication number: WO 2019/240008

(57) **Abstract**

Provided is an information processing apparatus including an acquiring unit that acquires cell information indicating a state of a cell from production of a pluripotent stem cell to differentiation of the pluripotent stem cell into a specific differentiated cell by differentiation induction, and process history information indicating a history of a processing process for obtaining the differentiated cell, and a deriving unit that derives differentiation potency information indicating differentiation potency of the pluripotent stem cell based on the cell information and the process history information which are acquired by the acquiring unit.

## Description

### Technical Field

This application claims priority to Japanese Patent Application No. 2018-0112878 filed on June 13, 2018, incorporated herein by reference in its entirety.

The disclosed technique relates to an information processing apparatus, a derivation method of deriving information regarding a differentiated cell, and a derivation program.

### Background Art

The following techniques are known as techniques for estimating the characteristics of cells to be evaluated. For example, JP2011-229410A discloses a cell evaluating apparatus comprising an image reading unit that reads a plurality of images in which a plurality of cells cultured in a culture container are imaged in time series, a feature amount calculating unit that calculates a plurality of different feature amounts indicating a plurality of different morphological features from the images for each cell included in the image, a calculation model constructing unit that constructs a calculation model for evaluating characteristics of the cell.

WO2010/131641A discloses a technique for discriminating an undifferentiated state of a cell, a differentiated state of the cell, and resistance of the cell with respect to differentiation by using a supervised machine learning method or a semi-supervised machine learning method.

JP2017-009314A discloses an assistance method for creating of teaching data for machine learning of learning data used to classify an object from a shape of the object obtained by imaging a carrier that carries cells. The method comprises a display step of displaying a teaching image including a target object of teaching data creation on a display unit to enable classification of the target object, a data creating step of receiving a classification result of the object displayed on the display unit and creating the teaching data by associating the classification result with the teaching image.

### SUMMARY OF INVENTION

### Technical Problem

A pluripotent stem cell such as an induced pluripotent stem (iPS) cell and an embryonic stem (ES) cell is a cell potentially having potency of differentiation into various tissues of a living body, and can differentiate into endoderm, mesoderm, and ectoderm. The potency of the cell to differentiate into a different cell type is called differentiation potency. However, the pluripotent stem cell has a different differentiation potency for each clone, and in some cases, may not be able to differentiate into a specific cell. Such unevenness of the differentiation potency becomes apparent only after a differentiation induction process with respect to the pluripotent stem cell.

On the other hand, a relatively long period of time (for example, 3 months) is required from establishment of the pluripotent stem cell to obtaining of a cell after differentiation (hereinafter, referred to as a differentiated cell) such as endoderm, mesoderm, and ectoderm. In the production of regenerative medicine products derived from the pluripotent stem cell, the unevenness of the differentiation potency of the pluripotent stem cell is one factor that significantly reduces productivity. Therefore, it is considered that the productivity and the quality of the regenerative medicine products derived from the pluripotent stem cell can be improved in a case where the differentiation potency of the pluripotent stem cell can be estimated before the differentiated cell is obtained.

The disclosed technique has been made in view of the above circumstances, and provides an information processing apparatus that performs estimation of differentiation potency of a pluripotent stem cell before the pluripotent stem cell differentiates into a specific differentiated cell by differentiation induction, a derivation method, and a derivation program. Solution to Problem

An information processing apparatus according to an aspect of the disclosed technique comprises an acquiring unit that acquires cell information indicating a state of a cell from production of a pluripotent stem cell to differentiation of the pluripotent stem cell into a specific differentiated cell by differentiation induction, and process history information indicating a history of a processing process for obtaining the differentiated cell, and a deriving unit that derives differentiation potency information indicating differentiation potency of the pluripotent stem cell based on the cell information and the process history information that are acquired by the acquiring unit.

With the information processing apparatus according to the aspect of the disclosed technique, it is possible to perform estimation of the differentiation potency of the pluripotent stem cell before the pluripotent stem cell differentiates into a specific differentiated cell by the differentiation induction.

The deriving unit may derive the differentiation potency information by inputting the cell information and the process history information which are acquired by the acquiring unit to an estimation model. The estimation model may be a model learned by machine learning using, as learning data, a plurality of combinations of the cell information for learning, the process history information for learning, and type information for learning indicating a type of the differentiated cell obtained from the cell in the state indicated by the cell information for learning through the process history indicated by the process history information for learning.

Accordingly, it is possible to perform estimation of the differentiation potency of the pluripotent stem cell before the pluripotent stem cell differentiates into a specific differentiated cell by the differentiation induction.

The deriving unit may derive a score indicating a probability of differentiation into each of endoderm, mesoderm, and ectoderm as the differentiation potency information.

Accordingly, it is possible to quantitatively grasp the differentiation potency of the pluripotent stem cell.

The cell information may include at least one of appearance information regarding appearance of the pluripotent stem cell, gene information regarding a gene of the pluripotent stem cell, secretion information regarding a secretion secreted from the pluripotent stem cell, or donor information regarding a donor of a somatic cell used for establishing the pluripotent stem cell.

Accordingly, it is possible to improve the precision of estimation of the differentiation potency of the pluripotent stem cell.

The cell information may further include information regarding an undifferentiation-deviated cell in which the pluripotent stem cell deviates from an undifferentiated state before the differentiation induction.

Accordingly, it is possible to further improve the precision of estimation of the differentiation potency of the pluripotent stem cell.

The process history information may include at least one of operator information indicating an operator who performs an operation of the processing process, equipment information indicating equipment used in the processing process, identification information of a culture medium and a reagent used in the processing process, or environmental information indicating an environment of the processing process.

Accordingly, it is possible to improve the precision of estimation of the differentiation potency of the pluripotent stem cell.

A derivation method executed by a computer according to another aspect of the disclosed technique comprises acquiring cell information indicating a state of a cell from production of a pluripotent stem cell to differentiation of the pluripotent stem cell into a specific differentiated cell by differentiation induction, and process history information indicating a history of a processing process for obtaining the differentiated cell; and deriving differentiation potency information indicating differentiation potency of the pluripotent stem cell based on the acquired cell information and the acquired process history information.

A derivation program according to still another aspect of the disclosed technique causes a computer to execute acquiring cell information indicating a state of a cell from production of a pluripotent stem cell to differentiation of the pluripotent stem cell into a specific differentiated cell by differentiation induction, and process history information indicating a history of a processing process for obtaining the differentiated cell, and deriving differentiation potency information indicating differentiation potency of the pluripotent stem cell based on the acquired cell information and the acquired process history information.

### Effects of Invention

According to the disclosed technique, it is possible to perform estimation of the differentiation potency of the pluripotent stem cell before the pluripotent stem cell differentiates into a specific differentiated cell by the differentiation induction.

### BRIEF DESCRIPTION OF THE DRAWINGS

Fig. 1 is a process flow diagram showing an example of a rough flow of processing performed until differentiated cells such as endoderm, mesoderm, and ectoderm are obtained from a pluripotent stem cell.
Fig. 2 is a functional block diagram showing an example of a functional configuration of an information processing apparatus according to an embodiment of the disclosed technique in a learning phase.
Fig. 3 is a diagram showing an example of a structure of an estimation model according to an embodiment of the disclosed technique.
Fig. 4 is a functional block diagram showing an example of a functional configuration of an information processing apparatus according to an embodiment of the disclosed technique in an operation phase.
Fig. 5 is a diagram showing an example of a hardware configuration of the information processing apparatus according to the embodiment of the disclosed technique.
Fig. 6 is a flowchart showing an example of a flow of learning processing according to an embodiment of the disclosed technique.
Fig. 7 is a flowchart showing an example of a flow of differentiation potency estimation processing according to an embodiment of the disclosed technique.

### DESCRIPTION OF EMBODIMENTS

Hereinafter, embodiments of the present disclosure will be described with reference to drawings. In the drawings, substantially the same or equivalent constituent elements or parts are designated by the same reference numerals.

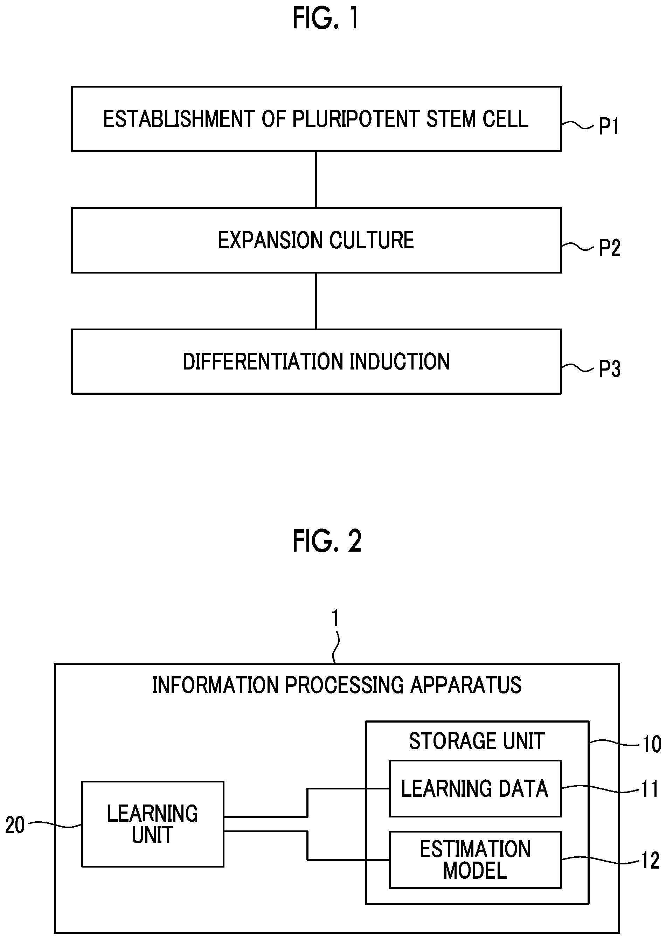

Fig. 1 is a process flow diagram showing an example of a rough flow of processing performed until differentiated cells (germ layer) such as endoderm, mesoderm, and ectoderm are obtained from a pluripotent stem cell.

In a first process P1, processing of establishing a pluripotent stem cell from a somatic cell collected from a living body is performed. For example, in a case where an iPS cell is used as the pluripotent stem cell, in the first process P1, processing of introducing reprogramming factors (Oct3/4, Sox2, c-Myc, and Klf4) into the somatic cell collected from the living body by using the episomal plasmid.

In a second process P2, expansion culture for growing the pluripotent stem cell. In the second process P2, the expansion culture is performed by an adhesion culture method in which the pluripotent stem cell is cultured on a flask coated with a matrix. In the expansion culture, culture medium exchange processing of exchanging a used culture medium with a fresh culture medium is performed at an appropriate time during the culture period. In addition, as the cells grow, processing of peeling and collecting the cell and seeding the cell again in a new flask (passaging process) is performed. The expansion culture is not limited to the adhesion culture, but a suspension culture method using a spinner flask can be adopted.

In a third process P3, differentiation induction is performed to differentiate the pluripotent stem cell into the differentiated cell (germ layer). In the third process P3, for example, processing of adding a differentiation inducer to the pluripotent stem cell. As the differentiation inducer, various growth factors/cytokines such as FGF-2, Activin, and BMP-4, and various signal inhibitors such as BMP signal inhibitor and Wnt signal inhibitor are used. Through the processes, the pluripotent stem cell usually differentiates into any one endoderm, mesoderm, and ectoderm. However, the differentiation potency of the pluripotent stem cell is uneven, there is the cell that does not differentiate into endoderm, mesoderm, and ectoderm.

An information processing apparatus according to the embodiment of the disclosed technique described below is to perform estimation of the differentiation potency of the pluripotent stem cell before the pluripotent stem cell differentiates into a specific differentiated cell (germ layer) by the differentiation induction. The information processing apparatus performs the above estimation using an estimation model. The estimation model is constructed by performing machine learning using learning data in a learning phase.

Fig. 2 is a functional block diagram showing an example of a functional configuration of an information processing apparatus 1 according to an embodiment of the disclosed technique in a learning phase.

The information processing apparatus 1 comprises a learning unit 20 and a storage unit 10. Learning data 11 and estimation model 12 are stored in the storage unit 10. An example of the contents of the learning data 11 is shown in Table 1. The learning data 11 is configured by a plurality of combinations of cell information for learning, process history information for learning, and differentiated cell information for learning. The learning data 11 is data used for learning of the estimation model 12 by machine learning of what kind of differentiated cell is obtained from the pluripotent stem cell in what kind of state and the biological cell used for establishment, through what kind of process history. Each of the learning data 11 is configured based on actual data obtained by experiment.

**[Table 1]**

| | |
|---|---|
| Cell information | Appearance information |
| | Gene information |
| | Secretion information |
| | Donor information |
| Process history information | Operator information |
| | Equipment information |
| | Identification information of culture medium and reagent |
| | Environmental information |
| Differentiated cell information | Type information |

The cell information is information indicating, from production (establishment) of a target pluripotent stem cell (hereinafter, referred to as a target stem cell) of differentiation potency estimation by the information processing apparatus 1 to the differentiation of the pluripotent stem cell into a specific differentiated cell (germ layer) by differentiation induction, a state of the target stem cell and the biological cell used for establishing the target stem cell. The cell information includes appearance information, gene information, secretion information, and donor information.

The appearance information is information regarding the appearance of the target stem cell. The appearance information includes, for example, the equivalent circle diameter, the circularity, and the nucleolar density of the target stem cell. These information items can be acquired from an image acquired by imaging the target stem cell in at least one process of the first process P1, the second process P2, or the third process P3.

The gene information is information regarding the gene of the target stem cell. The gene information includes an index value indicating a degree of undifferentiated maintenance of the target stem cell. An example of the index value indicating the degree of undifferentiated maintenance of the target stem cell includes the positive rate of cell surface markers (SSEA-3 or Tra-1-60) or an amount of expression of undifferentiated marker genes (OCT3/4 or NANOG). The gene information is acquired by performing gene analysis on the target stem cell extracted in at least one process of the first process P1, the second process P2, or the third process P3.

The secretion information is information regarding the secretion secreted from the target stem cell. The secretion information includes, for example, the concentration of the secretion such as lactate secreted from the target stem cell in the second process P2.

The donor information is information regarding a donor of the somatic cell used for establishing the target stem cell. The donor information includes, for example, race, sex, and age of the donor of the somatic cell.

The process history information is information indicating a process history of the processing process (the first process P1, the second process P2, and the third process P3) for obtaining the differentiated cell (germ layer). The process history information includes operator information, equipment information, identification information of a culture medium and a reagent, and environmental information.

The operator information is information indicating an operator who performs operation in each of the first process P1, the second process P2, and the third process P3.

The equipment information is information indicating equipment used in each of the first process P1, the second process P2, and the third process P3, and equipment operating conditions.

The identification information of the culture medium and the reagent is identification information of lot numbers of the culture medium and the reagent used in each of the first process P1, the second process P2, and the third process P3.

The environmental information is information indicating a state of the environment surrounding the target stem cell in each of the first process P1, the second process P2, and the third process P3. The environmental information includes, for example, a ambient temperature (air temperature), humidity, a culture medium temperature, oxygen concentration in the culture medium, and the cleanliness in the room.

The differentiated cell information is information regarding the differentiated cell obtained by differentiation induction. The differentiated cell information includes type information. The type information is information indicating a type of the differentiated cell obtained from the pluripotent stem cell in the state indicated by the cell information through the process history indicated by the process history information (that is, whether it is endoderm, mesoderm, or ectoderm). The type information includes three scores corresponding to endoderm, mesoderm, and ectoderm. For example, in a case where the type of the obtained differentiated cell is endoderm, for example, the score corresponding to endoderm is 100, and the scores corresponding to mesoderm and ectoderm are 0, respectively. The differentiated cell information may include information regarding the target cell further differentiated from germ layer such as cardiomyocytes and nerve cells.

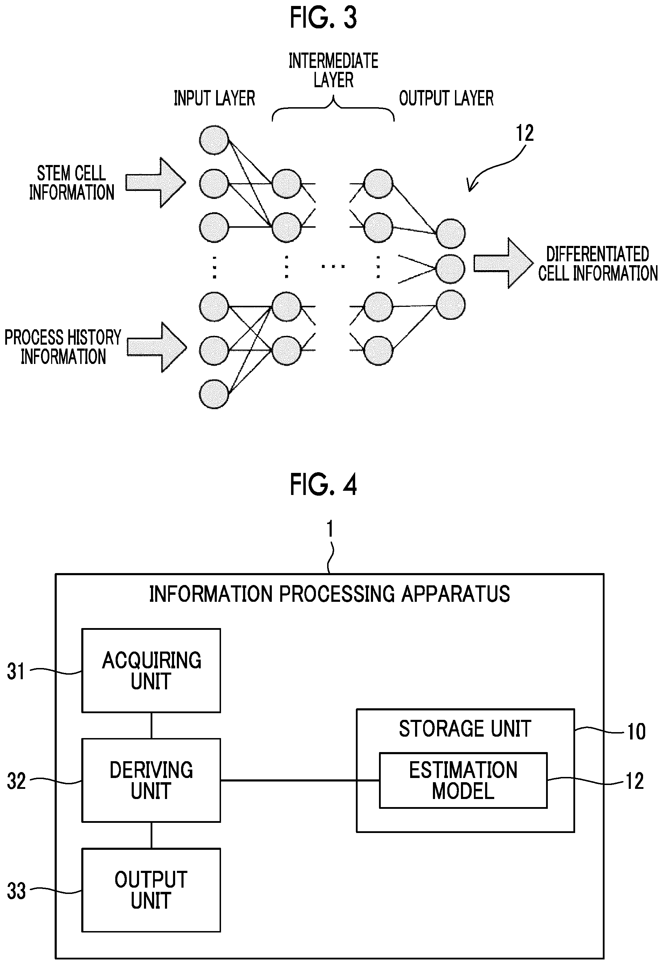

Fig. 3 is a diagram showing an example of the structure of the estimation model 12. The estimation model 12 is a neural network including an input layer, a plurality of intermediate layers, and an output layer. The estimation model 12 is a model for performing a virtual experiment, and one or more cell information items and one or more process history information items are input to the input layer of the estimation model 12. The index value indicating the differentiation potency of the target stem cell, which corresponds to a combination of the cell information and the process history information input to the input layer, is output from the output layer of the estimation model 12. The index value indicating the differentiation potency of the target stem cell corresponds to the type information included in the learning data 11, and includes three scores. One of the three scores indicates a probability of differentiation of the target stem cell into the endoderm, one of the three scores indicates a probability of differentiation of the target stem cell into the mesoderm, and one of the three scores indicates a probability of differentiation of the target stem cell into the ectoderm.

The learning unit 20 performs learning of the estimation model 12 according to a back propagation method as an example of machine learning, by using the learning data 11. Specifically, the learning unit 20 inputs the cell information for learning and the process history information for learning which are included in the learning data 11 to the estimation model 12, and acquires the index value (that is, three scores indicating the probabilities of differentiation into endoderm, mesoderm, and ectoderm) indicating the differentiation potency of the target stem cell output from the estimation model 12. The learning unit 20 performs learning of the estimation model 12 such that the difference between the acquired index value and the score indicated by the type information (differentiated cell information) for learning corresponding to the combination of the cell information and the process history information which are included in the learning data 11 is minimized.

The learning unit 20 performs processing of learning of the estimation model 12 by using all combinations of the cell information and the process history information which are included in the learning data 11, and the type information (differentiated cell information). The learning unit 20 may perform learning of the estimation model 12 by using a part of the combinations of the cell information and the process history information which are included in the learning data 11, and the type information (differentiated cell information).

Fig. 4 is a functional block diagram showing an example of a functional configuration of the information processing apparatus 1 according to the embodiment of the disclosed technique in an operation phase in which the learned estimation model 12 is operated to estimate the differentiation potency of the target stem cell. The information processing apparatus 1 comprises an acquiring unit 31, a deriving unit 32, an output unit 33, and the storage unit 10. The learned estimation model 12 constructed by performing machine learning in the learning phase is stored in the storage unit 10.

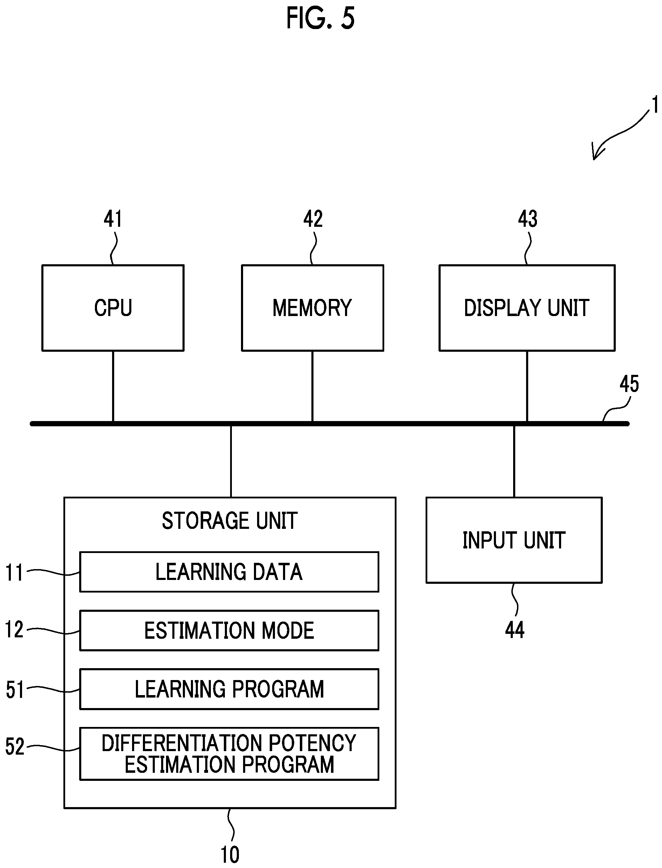

The acquiring unit 31 acquires the cell information and the process history information which are input by an input unit 44 (see Fig. 5).

The deriving unit 32 inputs the cell information and the process history information which are acquired by the acquiring unit 31 to the learned estimation model 12, and derives the index value indicating the differentiation potency of the target stem cell output from the estimation model 12 as the differentiation potency information. Specifically, the deriving unit 32 derives three scores indicating probabilities of differentiation of the target stem cell into endoderm, mesoderm, and ectoderm, as the differentiation potency information.

The output unit 33 outputs the differentiation potency information derived by the deriving unit 32 to a display unit 43 (see Fig. 5). The output unit 33 may output (store) the differentiation potency information derived by the deriving unit 32 to the storage unit 10.

Fig. 5 is a diagram showing an example of a hardware configuration of the information processing apparatus 1. The information processing apparatus 1 comprises a central processing unit (CPU) 41, a memory 42 as a temporary storage area, and the nonvolatile storage unit 10. The information processing apparatus 1 comprises the display unit 43 that is a display device such as a liquid crystal display, and the input unit 44 that is an input device such as a keyboard and a mouse. The CPU 41, the memory 42, the storage unit 10, the display unit 43, and the input unit 44 are connected via a bus 45.

The storage unit 10 is realized by, for example, a nonvolatile (non-transitory) storage medium such as a hard disk drive (HDD), a solid state drive (SSD), and the flash memory. A learning program 51, the learning data 11, the estimation model 12, and a differentiation potency estimation program 52 are stored in the storage unit 10. The CPU 41 develops the learning program 51 and the differentiation potency estimation program 52 in the memory 42, and then executes the programs. The CPU 41 functions as the learning unit 20 by executing the learning program 51. The CPU 41 functions as the acquiring unit 31, the deriving unit 32. and the output unit 33 by executing the differentiation potency estimation program 52.

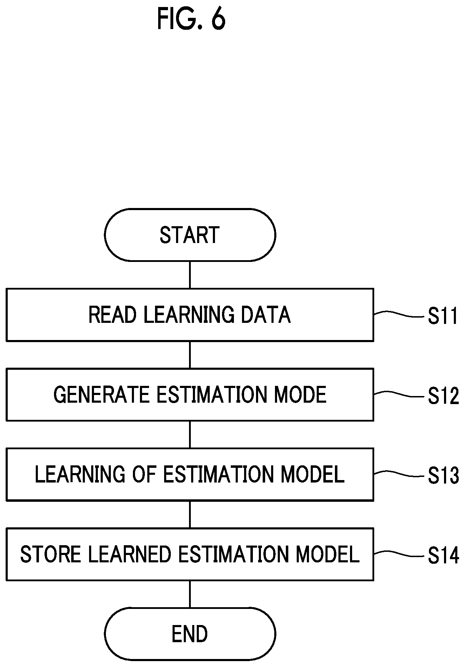

Fig. 6 is a flowchart showing an example of a flow of learning processing executed by the CPU 41 executing the learning program 51. For example, the CPU 41 executes the learning program in a case where the user inputs the execution instruction of the learning processing via the input unit 44 in the learning phase.

In step S11, the learning unit 20 reads the learning data 11 stored in the storage unit 10. The learning data 11 is configured by a plurality of combinations of the cell information for learning, the process history information for learning, and the type information (differentiated cell information) for learning.

In step S12, the learning unit 20 generates the estimation model 12. The parameters required for generating the estimation model, such as the number of nodes of the input layers of the estimation model 12, the number of layers and nodes of the intermediate layer, and an initial value of weights, are set by the user.

In step S13, the learning unit 20 performs learning of the estimation model 12 according to a back propagation method by using the learning data 11. Specifically, the learning unit 20 inputs the cell information and the process history information which are included in the learning data 11 to the estimation model 12, and acquires the index value (that is, three scores indicating the probabilities of differentiation into endoderm, mesoderm, and ectoderm) indicating the differentiation potency of the target stem cell output from the estimation model 12. The learning unit 20 performs learning of the estimation model 12 such that the difference between the acquired index value and three scores indicated by the type information (differentiated cell information) corresponding to the combination of the cell information and the process history information included in the learning data 11 is minimized.

In step S14, the learning unit 20 stores the learned estimation model 12 constructed by the processing of step S13 in the storage unit 10. In a case where the processing of step S14 is completed, the learning processing ends.

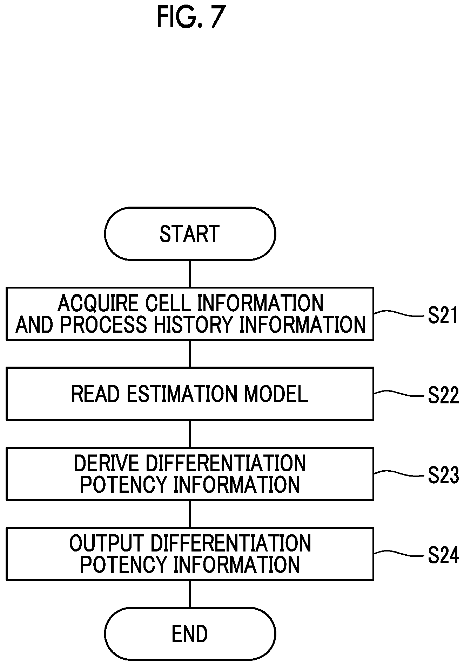

Fig. 7 is a flowchart showing an example of a flow of differentiation potency estimation processing executed by the CPU 41 executing the differentiation potency estimation program 52. For example, the CPU 41 executes the differentiation potency estimation program in a case where the user inputs the execution instruction of the differentiation potency estimation processing via the input unit 44 in the operation phase.

In step S21, the CPU 41 functions as the acquiring unit 31, and acquires the cell information and the process history information which are input by the input unit 44. The cell information and the process history information are acquired in at least one process of the first process P1, the second process P2, or the third process P3 shown in Fig. 1.

In step S22, the CPU 41 functions as the deriving unit 32, and reads the learned estimation model 12 from the storage unit 10.

In step S23, the CPU 41 functions as the deriving unit 32, inputs the cell information and the process history information which are acquired in step S21 to the learned estimation model 12, and derives the index value indicating the differentiation potency of the target stem cell output from the estimation model 12 as the differentiation potency information. Specifically, the CPU 41 functioning as the deriving unit 32 derives three scores indicating probabilities of differentiation of the target stem cell into endoderm, mesoderm, and ectoderm, as the differentiation potency information.

In step S24, the CPU 41 functions as the output unit 33, and outputs the differentiation potency information derived in the processing of step S23 to the display unit 43. In a case where the processing of step S24 is completed, the differentiation potency estimation processing ends.

As described above, with the information processing apparatus 1 according to the embodiment of the disclosed technique, the index value indicating the differentiation potency of the target stem cell is derived as the differentiation potency information by inputting the cell information and the process history information which are acquired in at least one process of the first process P1, the second process P2, or the third process P3 before the pluripotent stem cell differentiates. Specifically, three scores indicating probabilities of differentiation of the target stem cell into endoderm, mesoderm, and ectoderm is derived. Therefore, before the pluripotent stem cell differentiates, it is possible to grasp which of endoderm, mesoderm, and ectoderm the target stem cell has a relatively high probability of differentiating into. With the information processing apparatus 1 according to the embodiment of the disclosed technique, it is possible to perform estimation of the differentiation potency of the pluripotent stem cell before the pluripotent stem cell differentiates into a specific differentiated cell by the differentiation induction.

Therefore, depending on the estimated differentiation potency, it is possible to select a protocol for the processing performed in each process. For example, in a case where the information processing apparatus 1 estimates that the probability of differentiation of the target stem cell into the endoderm is relatively high, in the second process P2 and the third process P3, the processing protocol for inducing the differentiation into endoderm is selected and the yield and quality of the endoderm which is the differentiated cell can be improved.

With the information processing apparatus 1 according to the embodiment of the disclosed technique, in a case where the target stem cell has a high probability of no differentiating into any of endoderm, mesoderm, and ectoderm, all three scores derived as the differentiation potency information are low scores. In this case, the loss can be minimized by interrupting the processing. As described above, by utilizing the differentiation potency information derived by the information processing apparatus 1 according to the embodiment of the disclosed technique, the subsequent policy is determined, and the productivity and the quality of the regenerative medicine product derived from the pluripotent stem cell can be improved.

In the present embodiment, a case is exemplified in which the cell information includes the appearance information, the gene information, the secretion information, and the donor information, but the cell information may include at least one of the appearance information, the gene information, the secretion information, or the donor information, or may include above described information items.

In the present embodiment, a case is exemplified in which the process history information includes the operator information, the equipment information, the identification information of the culture medium and the reagent, and the environmental information, but the process history information may include at least one of the operator information, the equipment information, the identification information of the culture medium and the reagent, or the environmental information, or may include information other than above described information items.

Various processing executed by the CPU executing the software (program) in the embodiment may be executed by various processors other than the CPU. As an example of the processor in this case include a programmable logic device (PLD) that is a processor of which circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC). Various processing may be executed by one of these various processors, or a combination of two or more processors of the same type or different types (for example, a plurality of FPGAs, or a combination of the CPU and the FPGA). The hardware structure of these various processors is, more specifically, an electric circuit in which circuit elements such as semiconductor elements are combined.

In the embodiment, an aspect is described in which the learning program 51 and the differentiation potency estimation program 52 are stored (installed) in the storage unit 10 in advance, but the invention is not limited thereto. The learning program 51 and the differentiation potency estimation program 52 may be recorded in the recording medium such as a compact disk read only memory (CD-ROM), a digital versatile disk read only memory (DVD-ROM), and a universal serial bus (USB) memory. In addition, the learning program 51 and the differentiation potency estimation program 52 may be downloaded from the external device via the network.

### [Second Embodiment]

Table 2 below shows an example of cell information according to a second embodiment of the disclosed technique. The cell information according to the present embodiment includes undifferentiation-deviated cell information. The undifferentiation-deviated cell is a pluripotent stem cell that unintentionally deviates from an undifferentiated state before the differentiation induction is performed in the third process P3. The undifferentiation-deviated cell information is considered to be specifically generated by the expression of a specific gene. The undifferentiation-deviated cell is information regarding the undifferentiation-deviated cell. For example, it is considered that the gene information of the undifferentiation-deviated cell has a correlation with the differentiation potency of the pluripotent stem cell established from a common route with the undifferentiation-deviated cell. Therefore, in the present embodiment, the cell information is configured to include the undifferentiation-deviated cell information.

**[Table 2]**

| | |
|---|---|
| Cell information | Appearance information |
| | Gene information |
| | Secretion information |
| | Donor information |
| | Undifferentiation-deviated cell |

That is, in the learning phase, the learning unit 20 inputs the cell information for learning including the undifferentiation-deviated cell information and the process history information which are included in the learning data 11 to the estimation model 12, and acquires the index value (that is, three scores indicating the probabilities of differentiation into endoderm, mesoderm, and ectoderm) indicating the differentiation potency of the target stem cell output from the estimation model 12. The learning unit 20 performs learning of the estimation model 12 such that the difference between the acquired index value and the score indicated by the type information (differentiated cell information) for learning corresponding to the combination of the cell information and the process history information which are included in the learning data 11 is minimized.

In addition, in the operation phase, the deriving unit 32 inputs the cell information including the undifferentiation-deviated cell information and the process history information to the learned estimation model 12, and derives the index value indicating the differentiation potency of the target stem cell output from the estimation model 12 as the differentiation potency information. Specifically, the deriving unit 32 derives three scores indicating probabilities of differentiation of the target stem cell into endoderm, mesoderm, and ectoderm, as the differentiation potency information.

As described above, with the information processing apparatus 1 according to the present embodiment, the undifferentiation-deviated cell information that is considered to have a correlation with the differentiation potency of the pluripotent stem cell is used for estimating the differentiation potency of the pluripotent stem cell, and thus the estimating precision of the differentiation potency can be further improved.

In the embodiments, as a hardware structure of a processing unit that executes various processing such as the learning unit 20, the acquiring unit 31, the deriving unit 32, and the output unit 33, various processors described below can be used. The various processors include, in addition to the CPU that is a general-purpose processor executing the software (program) and functioning as the various processing units, a programmable logic device (PLD) that is a processor of which circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of these various processors, or a combinations of two or more processors of the same type or different types (for example, a combination of a plurality of FPGAs, or a combination of the CPU and the FPGA). In addition, a plurality of processing units may be configured by one processor.

As an example of configuring a plurality of processing units with one processor, first, as represented by a computer such as a client computer or a server, there is a form in which one processor is configured by a combination of one or more CPUs and software, and the processor functions as a plurality of processing units. Second, as represented by a system on chip (SoC), there is a form in which a processor is used that realizes the functions of the entire system including a plurality of processing units with a single integrated circuit (IC) chip. As described above, the various processing units are configured by one or more of the above various processors as a hardware structure.

Furthermore, the hardware structure of these various processors is, more specifically, an electric circuit (circuitry) in which circuit elements such as semiconductor elements are combined.

## Claims

1. An information processing apparatus comprising:
an acquiring unit that acquires cell information indicating a state of a cell from production of a pluripotent stem cell to differentiation of the pluripotent stem cell into a specific differentiated cell by differentiation induction, and process history information indicating a history of a processing process for obtaining the differentiated cell; and
a deriving unit that derives differentiation potency information indicating differentiation potency of the pluripotent stem cell based on the cell information and the process history information that are acquired by the acquiring unit.

2. The information processing apparatus according to claim 1,
wherein the deriving unit derives the differentiation potency information by inputting the cell information and the process history information that are acquired by the acquiring unit to an estimation model, and
the estimation model is a model learned by machine learning using, as learning data, a plurality of combinations of the cell information for learning, the process history information for learning, and type information for learning indicating a type of the differentiated cell obtained from the cell in the state indicated by the cell information for learning through the process history indicated by the process history information for learning.

3. The information processing apparatus according to claim 1 or 2, wherein the deriving unit derives a score indicating a probability of differentiation into each of endoderm, mesoderm, and ectoderm as the differentiation potency information.

4. The information processing apparatus according to any one of claims 1 to 3, wherein the cell information includes at least one of appearance information regarding appearance of the pluripotent stem cell, gene information regarding a gene of the pluripotent stem cell, secretion information regarding a secretion secreted from the pluripotent stem cell, or donor information regarding a donor of a somatic cell used for establishing the pluripotent stem cell.

5. The information processing apparatus according to claim 4, wherein the cell information further includes information regarding an undifferentiation-deviated cell in which the pluripotent stem cell deviates from an undifferentiated state before the differentiation induction.

6. The information processing apparatus according to any one of claims 1 to 5, wherein the process history information includes at least one of operator information indicating an operator who performs an operation of the processing process, equipment information indicating equipment used in the processing process, identification information of a culture medium and a reagent used in the processing process, or environmental information indicating an environment of the processing process.

7. A derivation method executed by a computer, the method comprising:
acquiring cell information indicating a state of a cell from production of a pluripotent stem cell to differentiation of the pluripotent stem cell into a specific differentiated cell by differentiation induction, and process history information indicating a history of a processing process for obtaining the differentiated cell; and
deriving differentiation potency information indicating differentiation potency of the pluripotent stem cell based on the acquired cell information and the acquired process history information.

8. A derivation program causing a computer to execute:
acquiring cell information indicating a state of a cell from production of a pluripotent stem cell to differentiation of the pluripotent stem cell into a specific differentiated cell by differentiation induction, and process history information indicating a history of a processing process for obtaining the differentiated cell; and
deriving differentiation potency information indicating differentiation potency of the pluripotent stem cell based on the acquired cell information and the acquired process history information.
